## Europäisches Patentamt

(19) **European Patent Office**

**Office européen des brevets**

(11) Numéro de publication: **0 161 645**
**B1**

(12) **FASCICULE DE BREVET EUROPEEN**

(45) Date de publication du fascicule du brevet:
**27.01.88**

(21) Numéro de dépôt: **85105758.8**

(22) Date de dépôt: **10.05.85**

(51) Int. Cl.⁴: **A 61 F 9/00,** A 61 B 3/00

(54) **Verre de contact pour l'ophtalmoscopie et l'ophtalmotherapie par rayons laser.**

(30) Priorité: **17.05.84 FR 8407835**

(43) Date de publication de la demande:
**21.11.85 Bulletin 85/47**

(45) Mention de la délivrance du brevet:
**27.01.88 Bulletin 88/4**

(84) Etats contractants désignés:
**CH DE GB LI**

(56) Documents cités:
**EP - A - 0 059 159**
**EP - A - 0 092 513**
**FR - A - 2 304 315**
**US - A - 4 033 679**

(73) Titulaire: **LASAG AG, Steffisburgstrasse 1,**
**CH-3600 Thun (CH)**

(72) Inventeur: **Rol, Pascal, Résidence du Lac**
**Staatstrasse 73, CH-3654 Gunten (CH)**

(74) Mandataire: **Gresset, Jean et al, ICB Ingénieurs Conseils**
**en Brevets SA Passage Max. Meuron 6,**
**CH-2001 Neuchâtel (CH)**

## Description

La présente invention concerne un verre de contact pour l'observation de l'intérieur de l'oeil et son traitement par irradiation, principalement au niveau de la chambre antérieure.

L'oeil peut être le siège de différentes maladies atteignant, entre autres, le diaphragme, le cristallin ou l'apex de la chambre antérieure. Pour diagnostiquer l'affection, l'intérieur de l'oeil doit être inspecté optiquement de façon précise.

Diverses méthodes d'observation sont connues. Les images les meilleures sont obtenues lorsqu'il est fait usage d'un verre de contact appliqué sur l'oeil, permettant de déformer le moins posible le faisceau de lumière issu du point observé, ou point de travail, situé à l'intérieur de l'oeil. Comme exemple on peut citer les verres de contact de Goldmann et de Roussel. Le premier est décrit en particulier dans la publication intitulée «Gonioscopie und Goniofotographie» de Winfried Müller et Hans-Peter Brandt, édition Ferdinand Enke, Stuttgart 1979, et le second dans la demande de brevet européen EP-A-0 059 159 au nom de la demanderesse.

Le verre de Goldmann présente au faisceau lumineux incident une face d'entrée plane et une face de sortie sphérique destinée à être appliquée sur la cornée. Il peut aussi comporter une ou plusieurs faces ou parois réfléchissantes planes constituant des miroirs afin de permettre une observation indirecte de l'intérieur de l'oeil. Le verre de Roussel, de son côté, a une face d'entrée bombée, constituant une surface de phase pour le faisceau incident, et une face de sortie également sphérique.

Les médecins opthalmologues apprécient beaucoup ces deux verres de contact pour l'observation étant donné leur maniabilité et le confort d'utilisation qu'ils apportent. Cependant la qualité de l'image qu'ils procurent est dégradée pour les faisceaux lumineux éloignés de l'axe optique de l'oeil.

Si le verre de contact est utile à l'observation de l'intérieur de l'oeil, il devient indispensable pour le traitement par irradiation de la chambre antérieure. Mais, travaillant alors dans des conditions différentes de celles de l'observation, il doit répondre à des exigences plus sévères.

Le traitement de l'intérieur de l'oeil par irradiation est en effet obtenu, en focalisant sur la région malade ou une partie de cette région, un intense faisceau de lumière cohérente. Ce faisceau peut être fourni par un laser Nd-YAG ou Argon par exemple.

Pour rendre le traitement efficace et sans danger, il est nécessaire que le faisceau atteignant le point de travail soit très bien focalisé et fortement convergent ou ouvert, c'est-à-dire que l'angle formé par les rayons lumineux extrêmes soit grand. La condition de bonne focalisation est évidente car elle permet de localiser avec précision le point de travail et d'y concentrer une énergie importante. La condition de forte convergence renforce la précédente. En effet, elle permet de bien localiser le point de travail en profondeur. D'autre part, la densité d'énergie du faisceau diminue alors rapidement en dehors du point de travail, ce qui a pour conséquence de réduire le danger de lésion des parties saines de l'oeil. La focalisation du faisceau lumineux sera d'autant meilleure que les défauts d'aberration sphérique et d'astigmatisme du système optique qu'il traverse, constitués par le verre de contact et l'oeil, seront faibles, Or généralement ces défauts augmentent avec le diamètre ou la convergence du faisceau incident. Ces deux conditions sont donc contradictoires et un compromis doit être trouvé pour chaque utilisation particulière. Le verre de Goldmann ne résous pas de façon satisfaisante le problème lié à l'ouverture du faisceau lumineux et encore moins celui qui concerne l'astigmatisme.

Diverses améliorations ont été apportées au verre de Goldmann. Des versions plus performantes de ce verre sont ainsi connues, par exemple le verre de Roussel, déjà cité, développé plus récemment. La face d'entrée non-plane de ce verre permet en effet de diminuer les aberrations et d'augmenter l'ouverture du faisceau.

Il faut aussi relever que toutes les tentatives d'amélioration du verre de Goldmann ont été faites en considérant le verre de contact pour lui-même, sans tenir compte des propriétés optiques de l'oeil qu'il était destiné à observer ou à irradier. Ainsi, un verre de contact ne donnant pas de défaut ne permettrait pas de focaliser parfaitement un faisceau lumineux en un point de la chambre antérieure éloigné de l'axe de l'oeil. En effet, pour atteindre ce point, le faisceau doit traverser différentes parties de l'oeil et, en particulier, la cornée qui donne un défaut d'astigmatisme d'autant plus grand qu'elle est vue sous un angle d'incidence élevé. Ceci a pour effet de détériorer le faisceau. Or, aucun verre de contact, du type Goldmann ou autre, ne tient compte de ce phénomène, ce qui représente un défaut très gênant des verres connus destinés au traitement de la chambre antérieure en des points situés hors de l'axe de l'oeil.

L'objet principal de l'invention est de fournir un verre de contact destiné à l'examen et au traitement par irradiation des différentes parties internes de l'oeil principalement de la chambre antérieure, ne présentant pas ces inconvénients.

Pour atteindre cet objectif, le verre de contact selon l'invention pour l'observation et le traitement de l'oeil par un faisceau lumineux, comprenant un élément principal pourvu d'une face d'entrée par laquelle pénètre le faisceau, d'une face de sortie, destinée à être appliquée sur la cornée de l'oeil, par laquelle sort le faisceau vers un point de travail à l'intérieur de l'oeil, et d'une face réfléchissante ayant pour fonction de dévier par une réflexion totale le faisceau quittant la face d'entrée vers la face de sortie, est particulièrement remarquable en ce qu'il comprend, en outre, un élément de compensation, associé à la face réfléchissante, dont la fonction est de créer dans le verre de contact un défaut d'astigmatisme inverse de celui de l'oeil.

Un avantage du verre de contact selon l'invention est de permettre la focalisation précise d'un faisceau lumineux incident en un point de la chambre antérieure, malgré les défauts optiques des parties de l'oeil traversées par ce faisceau.

D'autres avantages ressortiront de la description qui sera faite maintenant à l'aide du dessin annexé qui illustre, à titre d'exemple nullement limitatif, un verre de contact selon l'invention. Sur ce dessin où les mêmes éléments portent les mêmes références:

la figure 1 montre, en coupe selon un plan de symétrie longitudinal, une représentation schématique de l'oeil sur la cornée duquel est appliqué un verre de contact de Goldmann déjà cite; et

les figures 2a à 2c représentent des exemples de réalisation du verre de contact selon l'invention.

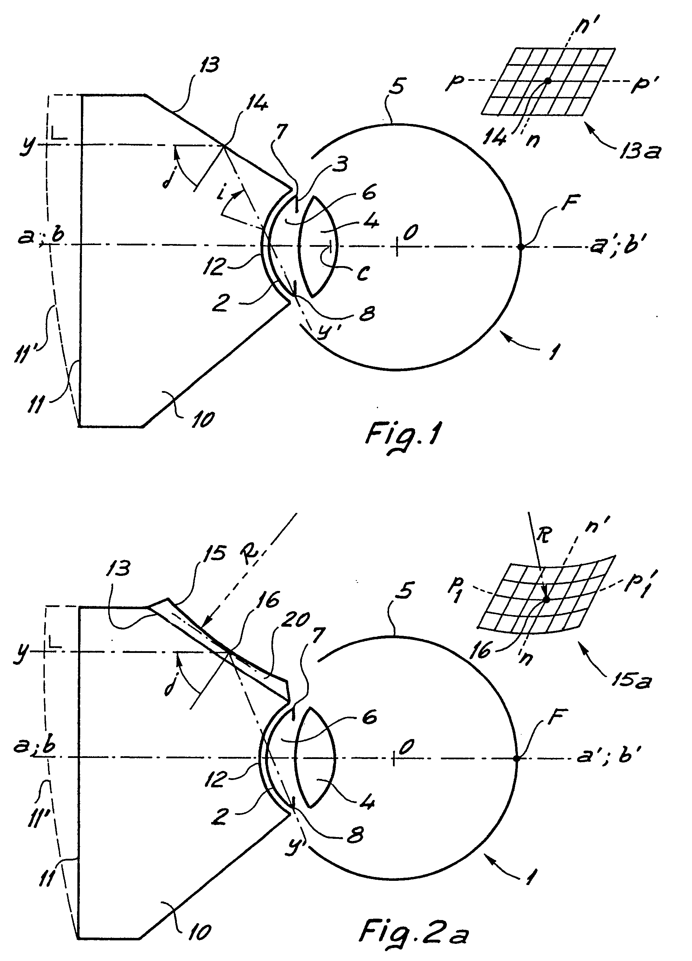

Une représentation schématique de l'oeil 1 est montrée en coupe longitudinale selon un plan de symétrie sur la figure 1. C'est un corps de révolution ayant un axe de symétrie aa' qui comprend, en allant de l'extérieur vers l'intérieur de l'oeil, une cornée sphérique 2, ayant le point C comme centre de courbure, par laquelle entrent les rayons lumineux, un iris 3 dont l'ouverture règle la quantité de lumière, un cristallin 4 et une rétine 5, de forme sphérique ayant un centre 0, qui est traversée par l'axe aa' en un point F. La cornée 2 et le cristallin 4 délimitent un volume référence 6, appelé chambre antérieure. Le lieu de rencontre de la cornée 2 et de l'iris 3 définit un cercle 7 dont chaque point, comme par exemple le point référencé 8, est appelé apex.

Pour bien comprendre la présente invention, il est utile de décrire d'abord la structure du verre de contact de Goldmann à miroir, déjà connu de l'art antérieur, et aussi représenté en coupe sur la figure 1. Ce verre de forme générale conique, référencé 10, comprend une face d'entrée plane 11 formant la buse du cône, une face de sortie sphérique 12 de centre de courbure C situé près du sommet du cône, et une face réfléchissante ou miroir plan 13 usiné sur la face latéral du cône. La face de sortie est appliquée sur la cornée 2. Les rayons lumineux subissent sur la face réfléchissante une réflexion totale. Une partie de ce miroir est schématiquement représentée en perspective en 13a où l'axe pp' est une droite du plan de la figure 1 et l'axe nn' une droite perpendiculaire à ce plan. D'autres faces réfléchissantes, non représentées, peuvent être répartis sur le pourtour du verre. L'angle entre une face réfléchissante et la face d'entrée dépend du point visé et il peut varier entre 50° et 80°. La droite perpendiculaire à la face d'entrée 11 et passant par le centre de courbure C de la face de sortie 12 définit l'axe de symétrie bb' du verre de contact 10. Dans le cas de la figure 1, les axes aa' et bb' sont confondus. Les dimensions du verre 10 permettent son basculement sur la cornée 2 de façon à pouvoir déplacer, dans certaines limites, le point d'observation ou de traitement, dit aussi point de travail, à l'intérieur de l'oeil. L'axe bb' pivote alors autour du centre de courbure C commun à la cornée 2 et à la face de sortie 12.

Le verre de Roussel a une forme générale cylindrique, sa section est presque identique à celle du verre de Goldmann représentée sur la figure 1. Il comporte ainsi une face de sortie 12 et une face réfléchissante 13. Par contre, sa face d'entrée a la forme d'une calotte sphérique, référencée 11', au lieu d'être plane. Le centre de courbure, non représenté, de la calotte sphérique est situé du côté de la face de sortie 12 et décentré vers la face réfléchissante 13 par rapport à l'axe bb'.

Le domaine d'application du verre de Goldmann est assez étendu puisque pratiquement tous les points essentiels de l'intérieur de l'oeil lui sont accessibles, aussi bien pour l'observation que pour le traitement.

Le but de la présente invention étant de fournir un verre de Goldmann ou de Roussel modifié qui permette d'observer et surtout de traiter par irradiation tout point de la chambre antérieure 6 dans de bonnes conditions, la figure 1 montre le cheminement de la lumière dans un verre de Goldmann lorsque le point d'apex 8 est visé. Pour atteindre ce point 8, un faisceau de lumière Y, dont seul l'axe yy' a été représenté, doit, après son entrée dans le verre 10 perpendiculairement à la face 11, ou à la face 11' dans le cas du verre de Roussel, subir une réflexion totale par la face 13 en un point 14 afin de pénétrer dans l'oeil 1 sous un angle d'incidence i mesuré par rapport à la normale à la face de sortie 12 en ce point. La réflexion totale fait que la face 13 n'a pas besoin d'être recouverte par une couche opaque réfléchissante.

L'oeil n'étant pas un système optique parfait, même un verre de contact ne donnant pas de défaut ne permettra pas de focaliser un faisceau lumineux en un point, mais seulement au mieux dans un cercle de diffusion de diamètre plus ou moins réduit. En effet, l'oeil présente un défaut d'astigmatisme d'autant plus prononcé que l'angle d'incidence i du faisceau lumineux traversant la cornée est élevé. Pour obtenir une focalisation plus précise, le verre de contact doit donc présenter un défaut d'astigmatisme inverse de celui de l'oeil.

Il existe plusieurs modèles de l'oeil, par exemple celui de Gullstrand-Legrand ou celui de Littmann, permettant de décrire avec précison ses propriétés optiques. Il est ainsi bien connu que la surface de l'oeil, comme toute surface optique sphérique, donne, lorsqu'elle est traversée par un faisceau incident, un défaut d'astigmatisme qui peut être décomposé en une partie sagittale et une partie tangentielle. Chaque partie est définie par sa distance focale, mesurée, par exemple, à partir de la face d'entrée de l'oeil. La distance focale correspondant à la partie sagittale est désignée par S et celle correspondant à la partie tangentielle par T.

Des considérations théoriques et des essais ont montré qu'il est possible d'obtenir un verre de contact compensé présentant le défaut d'astigmatisme inverse voulu en associant à la face réfléchissante 13 d'un verre de Goldmann un élément de compensation de forme simple, facile et peu coûteux à réaliser.

L'élément de compensation peut modifier le point de visée du verre de Goldmann. Cette modification reste cependant faible et elle peut être compensée par un léger changement, de quelques degrés au maximum, de l'inclinaison de la face réfléchissante. Pratiquement, il est toujours possible de fabriquer ou de trouver, pour chaque point de visée, un verre du type de Goldmann adéquat. En effet, il existe des verres standards dont l'inclinaison de la face réfléchissante, par rapport à la face d'entrée, est comprise entre 50° et 80°.

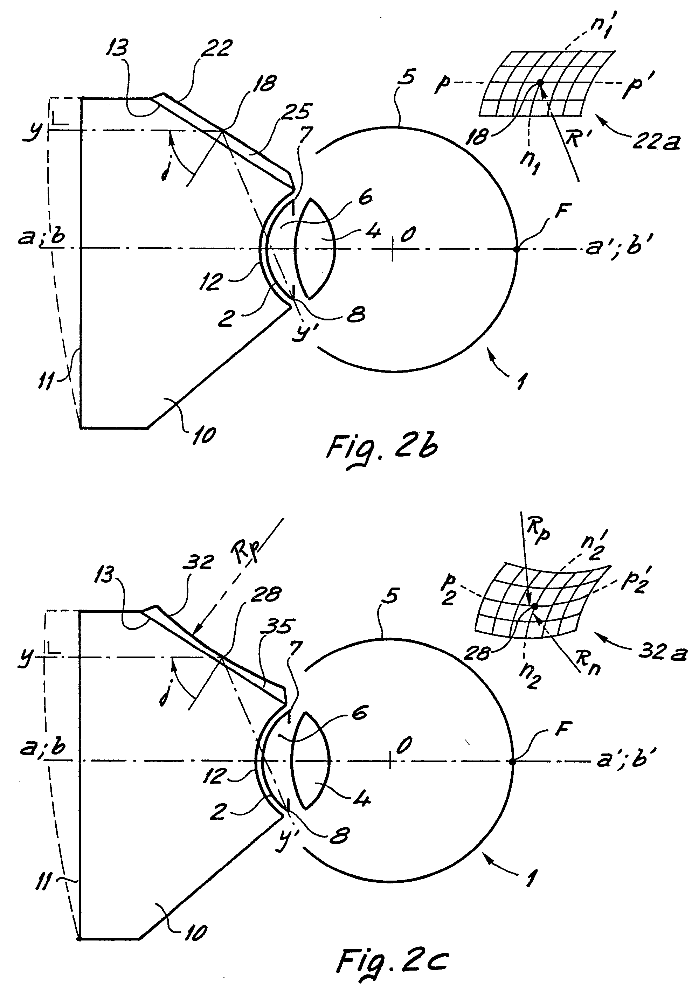

Une première forme d'exécution d'un verre de contact compensé selon l'invention est représentée sur la figure 2a en coupe longitudinale selon un plan de symétrie du verre. Dans cet exemple, l'élément de compensation est une lentille plano-cylindrique 20 dont l'axe est perpendiculaire au plan du dessin. Cette lentille, présentant une face plane et une face cylindrique 15, est orientée de façon que sa face plane soit appliquée sur la face réfléchissante 13 du verre de Goldmann 10. La face cylindrique 15 de la lentille 20 a un rayon de courbure R, le centre de courbure étant situé du même côté que la face 15 par rapport à la face 13. Une partie de la face 15 est montrée en perspective en 15a où pp' représente un arc de cercle situé dans le plan de la figure 2a et nn' une droite perpendiculaire à ce plan.

Sur la figure 2a est également représenté le trajet d'un faisceau lumineux d'axe yy' aboutissant au point d'apex 8 dans le cas où le verre de Goldmann 10 et le prisme 20 sont réalisés dans le même matériau. Ce faisceau, pénétrant dans le verre de Goldmann 10 perpendiculairement à la face 11, traverse la face 13 sans subir de déviation, les matériaux de part et d'autre de cette face étant identiques, pour atteindre la face cylindrique 15 de la lentille 20 en un point 16 sous un angle d'incidence j. Après avoir subi une réflexion totale au point 16, le faisceau quitte la face 15 sous le même angle j pour pénétrer dans la chambre antérieure 6 qu'il traverse jusqu'à l'apex 8.

Bien entendu, étant donné que la face 15 est cylindrique, l'angle j varie avec la position du point 16, lequel dépend du point d'impact du faisceau yy' sur la face 11. Cependant, comme le rayon de courbure R est pratiquement très grand par rapport aux dimensions de la lentille 20, les déplacements limités que peut subir le faisceau yy' n'entraînent que des variations très faibles de l'angle j, qui sera considéré comme étant constant.

Dans la forme d'exécution du verre de contact compensé représentée sur la figure 2a, l'élément de compensation est la lentille plano-cylindrique 20. Or il est bien connu qu'une telle lentille présente, pour le faisceau lumineux d'axe yy', un défaut d'astigmatisme. Cet astigmatisme dépend de l'angle j, de la distance e, non représentée sur le dessin, que parcourt le faisceau yy' entre la face 15 et la face 12 et du rayon de courbure R de la face 15.

Si $R = 2(T+e)(S+e) / [(T-S) \cos j]$, S et T étant, comme déjà mentionné, les distances focales correspondant respectivement aux parties sagittale et tangentielle de l'astigmatisme, alors l'astigmatisme de la lentille 20 devient exactement inverse de celui de l'oeil, réalisant ainsi la correction voulue du verre de Goldmann. Typiquement R vaut environ 3.50 m.

Bien entendu, le verre de contact corrigé de la figure 2a peut être fait d'une seule pièce. D'autre part, il est évident que seule la partie de la lentille 20 qui reçoit le faisceau de lumière d'axe yy' est fonctionnelle. Le reste de la lentille peut avoir une forme quelconque.

Une deuxième forme d'exécution, utilisant une lentille plano-cylindrique 25 comme élément de compensation, est représentée sur la figure 2b. Cette lentille a une face plane et une face réfléchissante cylindrique 22. Elle est placée de façon que sa face plane soit appliquée sur la face 13 du verre de Goldmann 10 et que l'axe de la face 22 se trouve dans le plan du dessin. La face 22 a un rayon de courbure R', le centre de courbure étant situé du même côté que la face 13. Une partie de la face cylindrique 22 a été représenté en perspective en 22a où pp' est une droite située dans le plan de la figure 2b et $n_1 n_1'$ un arc de cercle se trouvant dans un plan perpendiculaire au précédent. Enfin, le trajet d'un faisceau lumineux d'axe yy' a également été représenté sur la figure 2b en supposant, comme dans le cas précédent, que le verre de Goldmann 10 et la lentille 25 sont faits d'un même matériau. Ce faisceau rencontre la face 22 en un point 18 sous un angle d'incidence j où une réflexion totale le dévie vers le point d'apex 8 en lui faisant parcourir la distance e entre les faces 22 et 12.

Pour que la lentille 25 présente un défaut d'astigmatisme exactement inverse de celui de l'oeil, le rayon de courbure R' de la face réfléchissante 22 doit avoir une valeur bien définie, donnée par la relation $R' = [2(T+e)(S+e) \cos j] / (S-T)$. Typiquement R' vaut 0.974 m.

Les relations donnant R et R' sont issues des formules d'optique, connues sous le nom d'équation de Coddington, figurant par exemple aux pages 186 et 187 de l'ouvrage «Lens Design Fondamentals» de Rudolph Kingslake, Academic Press, New-York, 1978.

Une troisième forme d'exécution est représentée sur la figure 2c. L'élément de compensation est, dans ce cas, une lentille plano-torique 35. Cette lentille a une face plane et une face réfléchissante torique 32. La face 32 est définie par deux rayons de courbure principaux Rp et Rn donnant des cercles dans deux plans perpendiculaires à partir de deux centres de courbures différents. Le premier centre de courbure, correspondant au rayon Rp, est situé du même côté que la face 32 par rapport à la face plane de la lentille 35, et le second centre de courbure, correspondant au rayon Rn, est situé du côté opposé à la face 32, par rapport à la même face plane. La lentille 35 est placée et orientée sur le verre de Goldmann 10 de manière que la face plane de la lentille vienne en contact avec la face 13 et que le plan du cercle décrit par le rayon Rp coïncide avec le plan du dessin. Une partie de la face 32 a été représentée en 32a où $p_2 p_2'$ représente un arc de cercle de rayon Rp situé dans le plan de la figure 2c et $n_2 n_2'$ un arc de cercle de rayon Rn situé dans un plan perpendiculaire au précédent.

En supposant, comme dans les cas précédents, que le verre de Goldmann 10 et la lentille 35 de la figure 2c sont faits d'un même matériau, un rayon lumineux d'axe yy' atteindra la face 32 en un point 28 pour y subir une réflexion totale le dirigeant vers le point d'apex 8.

Il est connu qu'une surface torique crée un défaut d'astigmatisme qui dépend des rayons Rp et Rn. Les équations de Coddington déjà citées permettent de déterminer ces rayons de façon que l'astigmatisme de la lentille 35 compense exactement celui de l'oeil. Plusieurs solutions sont possibles, par exemple Rp = 7.0 m et Rn = 1.93 m.

L'élément de compensation et le verre de Goldmann peuvent être réalisés dans le même matériau, sous forme de deux composants distincts. Ces deux composants, une fois réunis, permettent de définir physiquemet la face 13 dans le verre de contact ainsi obtenu. Cette face n'a cependant aucun effet sur les rayons lumineux, les indices de réfraction des deux côtés de cette face étant les mêmes. Le verre de contact compensé peut donc, tout aussi bien, être réalisé d'une seule pièce homogène dans laquelle la face 13 ne peut plus être localisée. Ce verre d'une seule pièce aura, bien entendu, les mêmes propriétés que le verre précédent. Des matériaux d'indice de réfraction différents peuvent évidemment aussi être utilisés pour l'élément de compensation et le verre de Goldmann. Les rayons du faisceau lumineux d'axe yy' subiront alors une première réfraction au niveau de la face 13 en entrant dans la lentille de compensation et, après avoir été réfléchi, une seconde refraction au niveau de la même face en entrant dans le verre de Goldmann. Bien entendu dans le calcul des rayons de courbure des faces réfléchissantes, il faudra tenir compte de cette modification du trajet des rayons lumineux.

Les moyens utilisés pour compenser le verre de Goldmann sont aussi applicables au verre de Roussel, les équations de Coddington, toujours valables, permettant de déterminer les rayons de courbure des lentilles.

Bien entendu, comme dans le cas du verre de Goldmann, la lentille de compensation et le verre de Roussel, peuvent être réalisés dans le même matériau ou des matériaux différents.

**Revendications**

1. Verre de contact pour l'observation et le traitement de l'oeil (1) par un faisceau lumineux (yy'), comprenant un élément principal (10) pourvu d'une face d'entrée (11, 11') par laquelle pénètre ledit faisceau, d'une face de sortie (12), destinée à être appliquée sur la cornée (2) de l'oeil, par laquelle sort ledit faisceau vers un point de travail (8) à l'intérieur de l'oeil, et d'une face réfléchissante (13) ayant pour fonction de dévier par une réflexion totale ledit faisceau quittant la face d'entrée vers la face de sortie, caractérisé en ce qu'il comprend, en outre, un élément de compensation (20, 25, 35), associé à ladite face réfléchissante, dont la fonction est de créer dans ledit verre un défaut d'astigmatisme inverse de celui de l'oeil.

2. Verre de contact selon la revendication 1, caractérisé en ce que ledit élément principal (10) est un verre de Goldmann et que ledit élément de compensation (20, 25, 35) crée ledit défaut d'astigmatisme pour un faisceau lumineux incident (yy') perpendiculaire à la face d'entrée (11) et frappant ledit verre du côté de ladite face réfléchissante (13).

3. Verre de contact selon la revendication 1, caractérisé en ce que ledit élément principal (10) est un verre de Roussel et que ledit élément de compensation (20, 25, 35) crée ledit défaut d'astigmatisme pour un faisceau lumineux incident (yy') perpendiculaire à la face d'entrée (11') et frappant ledit verre du côté de ladite face réfléchissante.

4. Verre de contact selon l'une des revendications 1, 2 ou 3, caractérisé en ce que ledit élément de compensation est une lentille plano-cylindrique (20, 25).

5. Verre de contact selon l'une des revendications 1, 2 ou 3, caractérisé en ce que ledit élément de compensation est une lentille plano-torique (35).

6. Verre de contact selon l'une des revendications précédentes, caractérisé en ce que ledit élément principal (10) et ledit élément de compensation (20, 25, 35) sont réunis en une seule pièce homogène.

**Patentansprüche**

1. Kontaktglas für die Beobachtung und die Behandlung des Auges (1) mittels eines Lichtbündels (yy'), bestehend aus einem Hauptelement (10) mit einer Eingangsfläche (11, 11'), durch die das genannte Lichtbündel eintritt, einer Ausgangsfläche (12), die auf die Augenhornhaut (2) aufgesetzt wird und durch die das genannte Bündel in Richtung auf einen Arbeitspunkt (8) im Innern des Auges austritt, und einer reflektierenden Fläche (13), deren Funktion es ist, das genannte, die Eingangsfläche verlassende Bündel durch eine Totalreflexion zur Ausgangsfläche abzulenken, dadurch gekennzeichnet, dass sie ferner ein der genannten reflektierenden Fläche zugeordnetes Kompensationselement (20, 25, 35) umfasst, deren Funktion darin besteht, in dem genannten Glas einen dem des Auges entgegengesetzten Astigmatismusfehler herbeizuführen.

2. Kontaktglas gemäss Anspruch 1, dadurch gekennzeichnet, dass das genannte Hauptelement (10) ein Goldmann-Glas ist und dass das genannte Kompensationselement (20, 25, 35) den genannten Astigmatismusfehler für ein senkrecht zur Eingangsfläche (11) einfallendes und das genannte Glas auf der Seite der genannten reflektierenden Fläche (13) treffendes Lichtbündel (yy') herbeiführt.

3. Kontaktglas gemäss Anspruch 1, dadurch gekennzeichnet, dass das genannte Hauptelement (10) ein Roussel-Glas ist und dass das genannte Kompensationselement (20, 25, 35) den genannten Astigmatismusfehler für ein senkrecht zur Eingangsfläche (11') einfallendes und das genannte Glas auf der Seite der genannten reflektierenden Fläche (13) treffendes Lichtbündel (yy') herbeiführt.

4. Kontaktglas gemäss einem der Ansprüche 1, 2 oder 3, dadurch gekennzeichnet, dass das genannte Kompensationselement eine planzylindrische Linse (20, 25) ist.

5. Kontaktglas gemäss einem der Ansprüche 1, 2 oder 3, dadurch gekennzeichnet, dass das genannte Kompensationselement eine plantorische Linse (35) ist.

6. Kontaktglas gemäss einem der vorstehenden Ansprüche, dadurch gekennzeichnet, dass das genannte Hauptelement (10) und das genannte Kompensationselement (20, 25, 35) in einem einzigen homogenen Stück vereint sind.

**Claims**

1. A contact lens for observation and treatment of the eye (1) by means of a light beam (yy'), comprising a principal element (10) provided with an entrance face (11, 11') by way of which the beam enters, an exit face (12) for application to the cornea (2) of the eye and by way of which the beam issues towards a working point (18) within the eye, a reflecting face (13) effective by total internal reflection to divert the beam entering at the entrance face towards the exit face, characterized in that it comprises a compensating element (20, 25, 35) which is associated with the reflecting face, the compensating element creating in the contact lens an astigmatism defect which is the reverse of that of the eye.

2. A contact lens according to claim 1, characterized in that the principal element (10) is a Goldmann lens and the compensating element (20, 25, 35) creates the astigmatism defect for an incident light (yy') beam which is perpendicular to the entrance face (11) and which impiges on the lens on the side of the reflecting face (13).

3. A contact lens according to claim 1, characterized in that the pincipal element (10) is a Roussel lens and the compensating element (20, 25, 35) creates the astigmatism defect for an incident light beam which is perpendicular to the entrance face (11) and which impiges on the lens on the side of the reflecting face.

4. A contact lens according to claim 1, 2, or 3, characterized in that the compensating element is a plano-cylindrical lens (20, 25).

5. A contact lens according to claim 1, 2 or 3, characterized in that the compensating element is a plano-toric lens (35).

6. A contact lens according to claims 1 to 5, characterized in that the principal element and compensating element are combined in a single homogenous piece.

Fig.1

Fig.2a

Fig. 2b

Fig. 2c